(19) Europäisches Patentamt
European Patent Office
Office européen des brevets

(11) EP 2 448 547 B1

(12) **EUROPÄISCHE PATENTSCHRIFT**

(45) Veröffentlichungstag und Bekanntmachung des Hinweises auf die Patenterteilung:
**14.03.2018 Patentblatt 2018/11**

(21) Anmeldenummer: **10723154.0**

(22) Anmeldetag: **17.06.2010**

(51) Int Cl.:
***A61K 8/31*** (2006.01) ***A61K 8/34*** (2006.01)
***A61K 8/81*** (2006.01) ***A61Q 5/06*** (2006.01)
***B05B 1/30*** (2006.01)

(86) Internationale Anmeldenummer:
**PCT/EP2010/058561**

(87) Internationale Veröffentlichungsnummer:
**WO 2011/000711 (06.01.2011 Gazette 2011/01)**

(54) **KOMPAKTES HAARSPRAY**
COMPACT HAIR SPRAY
SPRAY CAPILLAIRE COMPACT

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **01.07.2009 DE 102009031432**

(43) Veröffentlichungstag der Anmeldung:
**09.05.2012 Patentblatt 2012/19**

(73) Patentinhaber: **Henkel AG & Co. KGaA**
**40589 Düsseldorf (DE)**

(72) Erfinder:
- **BERGEMANN, Uwe**
**22459 Hamburg (DE)**
- **MÜLLER, Burkhard**
**21075 Hamburg (DE)**

(56) Entgegenhaltungen:
**EP-A1- 0 845 257** **WO-A2-2004/078810**
**WO-A2-2005/018588** **DE-A1-102004 051 647**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 448 547 B1

**Beschreibung**

Die Erfindung betrifft eine anwendungsfertig konfektionierte Zubereitung zur Behandlung keratinischer Fasern, insbesondere menschlicher Haare in Form eines Sprays sowie die Verwendung dieser Zubereitung zur Behandlung keratinischer Fasern, insbesondere menschlicher Haare.

Haarsprays enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden.

Bei treibgashaltigen Haarsprays werden flüchtige organische Verbindungen (so genannte VOC) emittiert. Es hat daher Versuche gegeben, vollständig wasserbasierte Sprays bereitzustellen, dabei entstand aber das Problem der geringeren Flüchtigkeit von Wasser im Vergleich zu beispielsweise den Alkoholen, was sich in längeren Trocknungszeiten auf dem Haar niederschlägt. Weiterhin ist aufgrund der häufig schlechteren Löslichkeit polymerer Verbindungen in wäßrigen Systemen diese Umstellung auch häufig mit dem Nachteil verbunden, daß beim Aufbringen der gewünschten Polymermenge auf das Haar Wasser zwangsläufig in solchen Mengen auf das Haar gelangt, daß die Trocknungszeiten unakzeptabel lang werden. Aus diesen Problemen heraus resultieren auch starke Schwankungen in der Dosierung der Mittel durch den Verbraucher.

Ein anderer Weg zur Verringerung des VOC-Ausstoßes besteht darin, die Haarsprays möglichst konzentriert bereitzustellen. Wegen der mit der Polymerkonzentration steigenden Viskosität der Zubereitungen treten jedoch verstärkt Probleme auf, ein gleichmäßiges und fein verteiltes Sprühbild zu erreichen, das seinerseits aber Voraussetzung für ein perfektes Styling ist. Die Abstimmung der Parameter eines Sprühsystems wie Art und Menge der festigenden Polymere, Viskosität der zu versprühenden Mischung, Art und Menge der Treibmittel sowie Material und Gestaltung der Sprüheinrichtung, stößt dabei zunehmend an Grenzen.

Aus der WO 2005/018588 A2 sind konzentrierte Kompakthaarsprays bekannt, welche mindestens 3,0 Gew.% mindestens eines filmbildenden und/oder festigenden Polymeren, mindestens 10,0 Gew.% eines protischen Lösemittels und 0 bis 98 Gew. % eines Treibmittels enthalten, wobei die mittlere Tröpfchengröße dieser Zusammensetzung beim Versprühen kleiner als 40 $\mu$m ist. In beispielhaften Zusammensetzungen werden Copolymere aus Acrylsäure, Ethylacrylat und N-tert.-Butylacrylamid eingesetzt, die geringe bis mittlere Molmassen aufweisen.

Es besteht daher weiterhin die Aufgabe, entsprechende hochkonzentrierte Mittel zu entwickeln, die hinsichtlich der anwendungstechnischen Eigenschaften die vom Verbraucher gesteckten Erwartungen erfüllen und dennoch weniger VOC emittieren als herkömmliche Produkte. Zusätzlich wird vom Verbraucher eine kleine Verpackung, welche sich bequem transportieren lässt und somit überall verfügbar ist, gewünscht. Trotz der gewünschten kleinen Verpackung soll jedoch der Inhalt für zahlreiche Anwendungen ausreichen und der Zahl der Anwendungen eines handelsüblichen Haarsprays entsprechen oder übertreffen.

Es wurde nun gefunden, daß sich spezielle Polymere mit höheren Molmassen mit Hilfe einer darauf abgestimmten Vorrichtung versprühen lassen und damit der VOC-Ausstoß verringert werden kann.

Gegenstand der vorliegenden Erfindung ist in einer ersten Ausführungsform eine Kombination aus einem treibmittelhaltigen Haarbehandlungsmittel und einem dafür geeigneten Spender, wobei

A) das treibmittelhaltige Haarbehandlungsmittel bezogen auf sein Gewicht

- a1) 7-13 Gew.-% eines Terpolymers aus N-Butylacrylamid, Ethylacrylat und Acrylsäure enthält, das einen K-Wert (gemessen in Anlehnung an DIN EN ISO 1628-1 bei 20°C, 1013,25 mbar in 1 Gew.-%iger Lösung des Polymers in Ethanol) von mindestens 35 aufweist,
- a2) mindestens 10 Gew.-% eines oder mehrerer protischer Lösungsmittel enthält,
- a3) 5 bis 60 Gew.-% Treibmittel enthält, und

B) der Spender ein Ventil umfaßt, welches

- b1) eine Ventilöffnung beinhaltet, die als Kegelbohrung ausgestaltet ist und deren Durchmesser 0,25 bis 0,3 mm beträgt,
- b2) eine Drosselung beinhaltet, welche einen Innendurchmesser von maximal 0,3 mm aufweist,
- b3) keine Seitenbohrung ("Gasphasenbohrung") aufweist.

In der erfindungsgemäßen Kombination wird eine Lösung eines Terpolymers aus N-Butylacrylamid, Ethylacrylat und Acrylsäure in protischem Lösungsmittel (Gemisch) mit Hilfe von Treibmittel(n) aus einer speziellen Vorrichtung versprüht. Die Abstimmung der Vorrichtung auf das hohe Molekulargewicht des Polymers führt zu einem feinen, gleichmäßigen Sprühbild mit geringer Tröpfchengröße. Durch den starken Halt, den das Polymer erzeugt, können geringere Mengen des Polymers pro Sprühstoß trealisiert werden, was gleichzeitig den Ausstoß von Treibmitteln verringert.

Die zu versprühenden Zusammensetzungen in der erfindungsgemäßen Kombination enthalten - bezogen af ihr Gewicht - 7-13 Gew.-% mindestens eines Terpolymers aus N-Butylacrylamid, Ethylacrylat und Acrylsäure. Das erfindungsgemäß eingesetzt Polymer weist dabei ein hohes Molgewicht auf, das sich in hohen K-Werten zeigt. Der K-Wert der Polymere wird im Rahmen der vorliegenden Erfindung in Anlehnung an die Norm DIN EN ISO 1628-1 bei 20°C, 1013,25 mbar in 1 Gew.-%iger Lösung des Polymers in Ethanol gemessen. Erfindungsgemäß weist das in der Kombination enthaltene eines Terpolymer aus N-Butylacrylamid, Ethylacrylat und Acrylsäure einen K-Wert von mindestens 35 auf.

Erfindungsgemäß besonders bevorzugte Kombinationen sind dadurch gekennzeichnet, daß das treibmittelhaltige Haarbehandlungsmittel ein Terpolymer aus N-Butylacrylamid, Ethylacrylat und Acrylsäure enthält, das einen K-Wert (gemessen in Anlehnung an DIN EN ISO 1628-1 bei 20°C, 1013,25 mbar in 1 Gew.-%iger Lösung des Polymers in Ethanol) von 35 bis 60, vorzugsweise von 35 bis 50 und insbesondere von 35 bis 45 aufweist.

Die besonders bevorzugte Terpolymere aus N-Butylacrylamid, Ethylacrylat und Acrylsäure mit K-Werten von 35 bis 45 sind kommerziell unter der Bezeichnung Ultrahold® strong (BASF) erhältlich. Ganz besonders bevorzugte erfindungsgemäße Kombinationen enthalten das Terpolymer im Haarbehandlungsmittel in engeren Grenzen. Hier sind bevorzugte Kombinationen dadurch gekennzeichnet, daß das treibmittelhaltige Haarbehandlungsmittel bezogen auf sein Gewicht 7 bis 13 Gew.-%, noch weiter bevorzugt 7,5 bis 12 Gew.-%, besonders bevorzugt 8 bis 11 Gew.-% und insbesondere 8,5 bis 10 Gew.-% mindestens eines Terpolymers aus N-Butylacrylamid, Ethylacrylat und Acrylsäure enthält. Zusätzlich zu dem erfindungsgemäß im treibmittelhaltigen Haarbehandlungsmittel enthaltenen Terpolymer kann das haarbehandlungsmittel in der erfindungsgemäßen Kombination weitere filmbildende und/oder festigende Polymere enthalten.

Selbstverständlich können auch mehrere filmbildende und/oder festigende Polymere in dem erfindungsgemäßen Mittel enthalten sein. Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt ein ionisches filmbildendes und/oder festigendes Polymer mit einem amphoteren und/oder nichtionischem filmbildenden und/oder festigenden Polymer gemeinsam verwendet. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft manche filmbildende Polymere beschrieben werden. Allerdings wird an dieser Stelle explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20%-iger wäßriger, alkoholischer oder wäßrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise, unter den Handelsbezeichnungen Ucon® der Union Carbide vertrieben werden. Besonders bevorzugt sind Polyvinylpyrrolidon und Polyvinylpyrrolidon/Vinylacetat Copolymere.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI® von der Firma Lehmann & Voss, Hamburg, vertrieben wird.

Festigende Polymere tragen zum Halt und/oder zum Aufbau des Haarvolumens, der Haarfülle der Gesamtfrisur bei. Diese sogenannten festigenden Polymere sind gleichzeitig auch filmbildende Polymere und daher generell typische Substanzen für Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares Feuchtigkeit, also Wasser zu absorbieren, verringern. Dadurch wird das schlaffe Herunterhängen der Haarsträhnen vermindert und somit wird ein langanhaltender Frisurenaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß ganz besonders bevorzugt zu verwendenden Polymere finden sich somit selbstverständlich gerade auch die kationischen Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid

Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Ganz besonders bevorzugt sind Acrylates/t-Butylacrylamide Copolymer, Octylacrylamide/Acrylates/Butylaminoethyl Methacrylate Copolymer, Polyurethane-1, Polyvinylcaprolactam und VP/VA Copolymer. Insbesondere bevorzugt wird ein den erfindungsgemäßen Mitteln eine Mischung aus Acrylates/t-Butylacrylamide Copolymer und Octylacrylamide/Acrylates/Butylaminoethyl Methacrylate Copolymer eingesetzt.

Die erfindungsgemäßen kationischen Polymere können sowohl festigende und/oder filmbildende und/oder antistatische und/oder avivierende Polymere als auch Polymere mit konditionierenden und/oder verdickenden Eigenschaften sein. Bei den geeigneten kationaktiven Polymeren handelt es sich vorzugsweise um haarfestigende und/oder um haarkonditionierende Polymere. Unter Polymeren sind sowohl natürliche als auch synthetische Polymere, welche kationisch oder amphoter geladen sein können, zu verstehen.

Bevorzugt sind solche Polymere, die eine ausreichende Löslichkeit in Wasser oder Alkohol besitzen, um in dem erfindungsgemäßen Mittel in vollständig gelöster Form vorzuliegen. Die kationische Ladungsdichte beträgt vorzugsweise 1 bis 7 meq/g.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen Luviquat® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid und das von der Firma ISP unter dem Handelsnamen Gafquat® HS 100 vertriebene Vinylpyrrolidon/Methacrylamidopropyltrimethylammoniumchlorid Copolymer.

Ein weiteres bevorzugtes kationische Polymer ist ein Homopolymer der allgemeinen Formel (C1-I),

$$
\begin{array}{ll}
R^1 & \\
| & \\
-[CH_2\text{-}C\text{-}]_n \qquad\qquad X^- & \qquad\qquad (C1\text{-}I) \\
| & \\
CO\text{-}O\text{-}(CH_2)_m\text{-}N^+R^2R^3R^4 &
\end{array}
$$

in der $R^1$= -H oder $-CH_3$ ist, $R^2$, $R^3$ und $R^4$ unabhängig voneinander ausgewählt sind aus C1-4-Alkyl-, -Alkenyl- oder-Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (C1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

$R^1$ steht für eine Methylgruppe
$R^2$, $R^3$ und $R^4$ stehen für Methylgruppen
m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (C1-I) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Geeignete kationaktive Silikonverbindungen weisen vorzugsweise entweder mindestens eine Aminogruppe oder mindestens eine Ammoniumgruppe auf. Geeignete Silikonpolymere mit Aminogruppen sind unter der INCI- Bezeichnung Amodimethicone bekannt. Hierbei handelt es sich um Polydimethylsiloxane mit Aminoalkylgruppen. Die Aminoalkylgruppen können seiten- oder endständig sein. Das N-haltige Silikon als erfindungsgemäßes kationisches Polymer (C1) kann vorzugsweise ausgewählt werden aus der Gruppe umfassend Siloxanpolymere mit wenigstens einer Aminogruppe, Siloxanpolymere mit wenigstens einer endständigen Aminogruppe, Amodimethicon, Trimethylsilylamodimethicone, und/oder Aminoethylaminopropylsiloxan-Dimethylsiloxan-Copolymer. Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen.

Erfindungsgemäß bevorzugt ist die Verwendung eines Aminosiloxans entsprechend der nachstehenden allgemeinen Formel (G1-II),

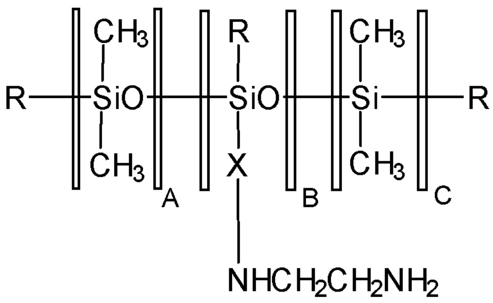

(G1-II)

wobei R = OH oder $CH_3$; X = Alkylgruppe mit 1 bis 4 C-Atomen, vorzugsweise Propyl oder Isopropyl und A, B und C = Copolymereinheiten, die taktische und/oder ataktische Polymerblöcke ausbilden können, sind.

Erfindungsgemäß am meisten bevorzugt ist Amodimethicon, Amodimethicon haltige Emulsionen oder Fluide. Emulsionen, die erfindungsgemäß bevorzugt eingesetzt werden können sind Dow Corning® 949, hierbei handelt es sich um eine kationische Emulsion enthaltend Amodimethicon, Cetrimoniumchlorid und Trideceth-12; Dow Corning® 939, hierbei handelt es sich um eine Emulsion enthaltend Amodimethicon, Cetrimoniumchlorid und Trideceth-12; Dow Corning® 929, hierbei handelt es sich um eine kationische Emulsion enthaltend Amodimethicon, Talktrimoniumchlorid und Nonoxynol-10; Dow Corning® 7224 oder 1401, basierend auf Trimethylsilylamodimethicon, Octoxynol-40, Isolaureth-6 und Glycol; Dow Corning® 2-8194 Mikroemulsion (26%ig) auf Basis eines aminfunktionalisierten Siliconpolymers; Dow Corning® 2-8177 Mikroemulsion (12%ig) auf Basis eines aminfunktionalisierten Siliconpolymers; Dow Corning® 2-8566 Amino Fluid auf Basis eines aminfunktionalisierten Polydimethylsiloxans; erhältlich bei der Firma Dow Corning.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 01, bis 0,5.

Das Silikon als kationisches Polymer wird in einer Menge von 0,01 bis 20 Gew.% bezogen auf das gesamte Mittel, bevorzugt in Mengen von 0,05 bis 15 Gew.% und ganz besonders bevorzugt in Mengen von 0,05 bis 10 Gew.% verwendet.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (G1-III) G-O-B-N+$R_aR_bR_c$ $X^-$

- G ist ein Anhydroglucoserest, beispielsweise Stärke- oder Celluloseanhydroglucose;
- B ist eine divalente Verbindungsgruppe, beispielsweise Alkylen, Oxyalkylen, Polyoxyalkylen oder Hydroxyalkylen;
- $R_a$, $R_b$ und $R_c$ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in $R_a$, $R_b$ und $R_c$ vorzugsweise maximal 20 ist;
- $X^-$ ist ein übliches Gegenanion und ist vorzugsweise Chlorid.

Eine kationische Cellulose wird unter der Bezeichnung Polymer JR® von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar® vertrieben und hat die INCI-Bezeichnung Guar Hydroxypropyltrimonium Chloride.

Besonders bevorzugte kationaktive Stoffe sind Chitosan, Chitosansalze und Chitosan-Derivate. Die Chitosanderivate sind ein Beispiel für einen kationisches Polymer, welches ausgeprägte Eigenschaften als Filmbildner hat. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200. 000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil& Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternierte, alkylierte oder hydroxyalkylierte Derivate, beispielsweise Hydroxyethyl- oder Hydroxybutylchitosan in Betracht. Weitere Chitosanderivate sind unter den Handelsbezeichnungen Hydagen® CMF, Hydagen® HCMF und Chitolam® NB/101 im Handel frei verfügbar.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gafquat®ASCP 1011, Gafquat®HS 110, Luviquat®8155 und Luviquat® MS 370 erhältlich sind.

Weitere geeignete kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Weiterhin können als Polymere amphotere Polymere verwendet werden. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefaßt, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen. Weiterhin geeignete zwitterionische Polymere sind Methacroylethylbetain/Methacrylat-Copolymere, die unter der Bezeichnung Amersette® (AMERCHOL) im Handel erhältlich sind.

Bevorzugt eingesetzte amphotere Polymere sind solche Polymerisate, die sich im wesentlichen zusammensetzen aus

(a) Monomeren mit quartären Ammoniumgruppen der allgemeinen Formel (G3-I),

$$R^1\text{-}CH\text{=}CR^2\text{-}CO\text{-}Z\text{-}(C_nH_{2n})\text{-}N^{(+)}R^3R^4R^5\ A^{(-)} \qquad \text{(G3-I)}$$

in der $R^1$ und $R^2$ unabhängig voneinander stehen für Wasserstoff oder eine Methylgruppe und $R^3$, $R^4$ und $R^5$

unabhängig voneinander für Alkylgruppen mit 1 bis 4 Kohlenstoffatomen, Z eine NH-Gruppe oder ein Sauerstoffatom, n eine ganze Zahl von 2 bis 5 und $A^{(-)}$ das Anion einer organischen oder anorganischen Säure ist, und

(b) monomeren Carbonsäuren der allgemeinen Formel (G3-II),

$$R^6\text{-CH=CR}^7\text{-COOH} \qquad \text{(G3-II)}$$

in denen $R^6$ und $R^7$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Schließlich kann die erfindungsgemäße Zusammensetzung insbesondere auf die festigende, avivierende und antistatische Wirkung gezielt beeinflusst werden, wenn anionische Polymere mit formuliert werden. Bei den anionischen Polymeren handelt es sich unter anderem um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.
Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Weiterhin erfindungsgemäß geeignete anionische Polymere sind u. a.:

- Vinylacetat/Crotonsäure-Copolymere, wie sie beispielsweise unter den Bezeichnungen Resyn® (NATIONAL STARCH), Luviset® (BASF) und Gafset® (GAF) im Handel sind.
- Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere.

Eine weitere ganz besonders bevorzugte Gruppe von Polymeren sind Polyurethane. Die Polyurethane bestehen

aus mindestens zwei verschiedenen Monomertypen,

- einer Verbindung (V1) mit mindestens 2 aktiven Wasserstoffatomen pro Molekül und
- einem Di- oder Polyisocyanat (V2).

Bei den Verbindungen (V1) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (V1) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und, -Diamine auf Basis von langkettigen Alkanen oder Polyalkylenoxiden.

Polyurethane, bei denen die Verbindungen (V1) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (V1) mit Dicarbonsäuren wie Phthalsäure, Isophthalsäure und Adipinsäure erhalten.

Als Verbindungen (V2) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fällen erfindungsgemäß besonders geeignet haben sich Polyurethane erwiesen, die wie folgt charakterisiert werden können:

- ausschließlich aliphatische Gruppen im Molekül
- keine freien Isocyanatgruppen im Molekül
- Polyether- und Polyesterpolyurethane
- anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Ein erfindungsgemäß ganz besonders bevorzugtes Polyurethan ist unter der Handelsbezeichnung Luviset® PUR (BASF) im Handel.

Die erfindungsgemäßen Mittel können in einer weiteren Ausführungsform nichtionogene Polymere enthalten.

Geeignete nichtionogene Polymere sind beispielsweise:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.

- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Weitere bevorzugte Polymere sind alle Polymere, welche im "International Cosmetic Ingredient Dictionary and Handbook", (seventh edition 1997, The Cosmetic, Toiletry, and Fragrance Association 1101 17th Street, N.W., Suite 300, Washington, DC 20036-4702) als Polymere in einem der Kapitel über Polymere wie beispielsweise "film formers" oder "hair fixatives" genannt und im Handel erhältlich sind. Auf diese Schrift und die daraus zitierten Abschnitte wird ausdrücklich Bezug genommen.

Es hat sich im Rahmen der vorliegenden Erfindung als vorteilhaft erwiesen, wenn weitere filmbildende und/oder festigende Polymere (d.h. solche, die nicht Terpolymere aus N-Butylacrylamid, Ethylacrylat und Acrylsäure sind und einen K-Wert (gemessen in Anlehnung an DIN EN ISO 1628-1 bei 20°C, 1013,25 mbar in 1 Gew.-%iger Lösung des Polymers in Ethanol) von mindestens 35 aufweisen) in den Haarbehandlungsmitteln der erfindungsgemäßen Kombination in Mengen von maximal 10 Gew.-%, bezogen auf das treibmittelhaltige Haarbehandlungsmittel, enthalten sind. In besonders bevorzugten erfindungsgemäßen Kombinationen sind im treibmittelhaltigen Haarbehandlungsmittel bezogen auf dessen Gewicht maximal 7,5 Gew.-%, weiter bevorzugt maximal 5 Gew.-%, noch weiter bevorzugt maximal 3 Gew.-%, noch weiter bevorzugt maximal 2,5 Gew.-% und insbesondere maximal 1,5 Gew.-% weiterer Filmbildender und/oder festigender Polymere enthalten.

In ganz besonders bevorzugten erfindungsgemäßen Kombinationen enthält das treibmittelhaltige Haarbehandlungsmittel außer dem bzw. den Terpolymer(en) aus N-Butylacrylamid, Ethylacrylat und Acrylsäure mit einen K-Wert (gemessen in Anlehnung an DIN EN ISO 1628-1 bei 20°C, 1013,25 mbar in 1 Gew.-%iger Lösung des Polymers in Ethanol) von mindestens 35) keine weiteren filmbildenden und/oder festigenden Polymere.

Das erfindungsgemäße Mittel wird bevorzugt in einem wässrigen, einem alkoholischen oder in einem wässrigalkoholischen Medium mit vorzugsweise mindestens 8, besonders bevorzugt mindestens 10 Gewichtsprozent Wasser konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Das erfindungsgemäße Mittel kann in einem pH-Bereich von 2 bis 11 vorliegen. Besonders bevorzugt ist der pH-Bereich zwischen 2 und 8.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent bevorzugt von 1 bis 10 Gewichtsprozent enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gewichtsprozent.

Zusammenfassend sind erfindungsgemäße Kombinationen bevorzugt, bei denen das treibmittelhaltige Haarbehandlungsmittel bezogen auf sein Gewicht 10 bis 80 Gew.-%, vorzugsweise 12,5 bis 70 Gew.-%, weiter bevorzugt 15 bis 65 Gew.-%, noch weiter bevorzugt 20 bis 60 Gew.-%, besonders bevorzugt 22,5 bis 55 Gew.-% und insbesondere 25 bis 50 Gew.-% mindestens eines protischen Lösungsmittels, ausgewählt aus Ethanol, Wasser und deren Mischungen, enthält.

Besonders bevorzugte erfindungsgemäße Kombinationen enthalten ein treibmittelhaltiges Haarbehandlungsmittel, das - bezogen auf sein Gewicht -

- 8 bis 10 Gew.-% Terpolymere aus N-Butylacrylamid, Ethylacrylat und Acrylsäure, die einen K-Wert (gemessen in Anlehnung an DIN EN ISO 1628-1 bei 20°C, 1013,25 mbar in 1 Gew.-%iger Lösung des Polymers in Ethanol) von mindestens 35 aufweisen, enthält
- 40 bis 50 Gew.-% Ethanol,
- 1 bis 5 Gew.-% Wasser

enthält.

Als weitere Komponente enthalten die erfindungsgemäßen Zubereitungen ist vorzugsweise ein Treibmittel.

Zur Anwendung der erfindungsgemäßen Zusammensetzungen als Aerosolsprays müssen Treibmittel verwendet werden. Die erfindungsgemäß bevorzugten Treibmittel sind ausgewählt aus den Kohlenwasserstoffen mit 3 bis 5 Kohlenstoffatomen, wie Propan, n-Butan, iso-Butan, n-Pentan und iso-Pentan, Dimethylether, Kohlendioxid, Distickstoffoxid, Fluorkohlenwasserstoffen und Fluorchlorkohlenwasserstoffen sowie Mischungen dieser Substanzen. Ganz besonders bevorzugte Treibgase sind Propan, Butan, Isobutan, Pentan, Isopentan, Dimethylether und die Gemische dieser zuvor genannten Treibgase jeweils untereinander. Erfindungsgemäß bevorzugteste Treibgase sind Dimethylether, Kohlenwasserstoffe und deren Gemische. Innerhalb der Gruppe der Kohlenwasserstoffe als Treibgasen bevorzugt sind n-Butan und Propan.

Vorteilhafterweise wird das Treibmittel so ausgewählt, daß es gleichzeitig als Lösungsmittel für weitere Inhaltsstoffe wie beispielsweise Öl- und Wachskomponenten, den Fettstoffen (D) dienen kann. Das Treibmittel kann dann als Lösungsmittel für diese letztgenannten Komponenten dienen, wenn diese bei 20 °C zu mindestens 0,5 Gew.-%, bezogen auf das Treibmittel, in diesem löslich sind.

Gemäß einer bevorzugten Ausführungsform enthalten die erfindungsgemäßen Zubereitungen die genannten Kohlenwasserstoffe, Dimethylether oder Mischungen der genannten Kohlenwasserstoffe mit Dimethylether als einziges Treibmittel. Die Erfindung umfaßt aber ausdrücklich auch die Mitverwendung von Treibmittel vom Typ der Fluorchlorkohlenwasserstoffe, insbesondere aber der Fluorkohlenwasserstoffe.

Ganz besonders bevorzugte erfindungsgemäße Kombinationen sind dadurch gekennzeichnet, daß das treibmittelhaltige Haarbehandlungsmittel bezogen auf sein Gewicht 7,5 bis 57,5 Gew.-%, vorzugsweise 10 bis 55 Gew.-%, weiter bevorzugt 15 bis 52,5 Gew.-%, noch weiter bevorzugt 17,5 bis 55 Gew.-%, besonders bevorzugt 20 bis 50 Gew.-% und insbesondere 25 bis 45 Gew.-% mindestens eines Treibmittels, ausgewählt aus n-Propan, n-Butan, iso-Butan, n-Pentan, Dimethylether und deren Mischungen, enthält.

Besonders bevorzugte erfindungsgemäße Kombinationen enthalten ein treibmittelhaltiges Haarbehandlungsmittel, das - bezogen auf sein Gewicht -

- 8 bis 10 Gew.-% Terpolymere aus N-Butylacrylamid, Ethylacrylat und Acrylsäure, die einen K-Wert (gemessen in Anlehnung an DIN EN ISO 1628-1 bei 20°C, 1013,25 mbar in 1 Gew.-%iger Lösung des Polymers in Ethanol) von mindestens 35 aufweisen,
- 40 bis 50 Gew.-% Ethanol,
- 1 bis 5 Gew.-% Wasser,
- 35 bis 45 Gew.-% Dimethylether

enthält.

Bei der Applikation der erfindungsgemäßen Zusammensetzung soll das Haar zu einer bestimmten Frisur geformt werden. Hierzu kann es erforderlich sein, die Haarstruktur und den Haarzustand positiv zu beeinflussen. Beispielsweise kann es vorteilhaft sein, wenn die Kämmwiderstände während des Aufbaues der Frisur niedrig sind. Weiterhin soll die gebildete Frisur einen gewissen Glanz oder farbig schimmernde Effekte zeigen. Das Haar soll im frisierten Zustand einen vitalen Eindruck hervorrufen. Daher ist es bevorzugt, pflegende Substanzen in die erfindungsgemäße Zusammensetzung einzuarbeiten.

Geeignete Verbindungen im Sinne der vorliegenden Erfindung als haarpflegende Verbindungen werden im Folgenden näher beschrieben.

Als erste Wirkstoffgruppe sind Fettstoffe (D) zu nennen. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen. Bevorzugt sind Fettsäuren mit 10 - 22 Kohlenstoffatomen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4), welche die Wirkung des erfindungsgemäßen Zusammensetzung steigern können, sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I),

$$\begin{array}{l} CH_2O(CH_2CH_2O)_mR^1 \\ | \\ CHO(CH_2CH_2O)_nR^2 \\ | \\ CH_2O(CH_2CH_2O)_qR^3 \end{array} \qquad (D4\text{-}I)$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäß verwendeten Mitteln beträgt üblicherweise 0,1 - 30 Gew.%, bezogen auf das gesamte Mittel, bevorzugt 0,1 - 20 Gew.-%, und insbesondere 0,1 - 15 Gew.-%.

Die Gesamtmenge an Öl- und Fettkomponenten in den erfindungsgemäßen Mitteln beträgt üblicherweise 0,1 - 50 Gew.-%, bezogen auf das gesamte Mittel. Mengen von 0,1 - 30 Gew.-% sind erfindungsgemäß bevorzugt.

Ebenfalls als vorteilhaft hat sich die Kombination der erfindungsgemäßen Kombination mit Tensiden (E) erwiesen. In einer weiteren bevorzugten Ausführungsform enthalten die erfindungsgemäß verwendeten Mittel Tenside. Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und - dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -$COO^{(-)}$ - oder - $SO_3^{(-)}$-Gruppe tragen. Besonders geeignete zwitterionische Tenside sind die sogenannten Betaine wie die N-Alkyl-N,N-dimethylammonium-glycinate. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das $C_{12}$- $C_{18}$ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch

solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die Tenside (E) werden in Mengen von 0,1 - 45 Gew.%, bevorzugt 0,1 - 30 Gew.% und ganz besonders bevorzugt von 0,1 - 20 Gew.%, bezogen auf das gesamte erfindungsgemäß verwendete Mittel, eingesetzt.

Ganz besonders bevorzugt sind erfindungsgemäß einsetzbar kationische Tenside (E5). Typische Beispiele für kationische Tenside sind insbesondere Tetraalkylammoniumverbindungen Amidoamine oder aber Esterquats. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid, Tricetylmethylammoniumchlorid, Hydroxyethyl Hydroxycetyl Dimmonium Chloride sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Kationische, nichtionische, zwitterionische und/oder amphotere Tenside sowie deren Mischungen können erfindungsgemäß bevorzugt sein.

Neben den zuvor beschriebenen kationischen Tensiden sind weiterhin besonders vorteilhaft kationaktive Polymere als haarpflegende Substanzen geeignet. Eine kationaktive Verbindung ist eine Substanz, die auf Grund von kationischen oder kationisierbaren Gruppen, insbesondere primären, sekundären, tertiären oder quaternären Amingruppen eine Substantivität zu menschlichem Haar aufweist. Geeignete kationaktive Stoffe sind ausgewählt aus kationischen Polymeren, Silikonverbindungen mit kationischen oder kationisierbaren Gruppen, kationisch derivatisierten Proteinen oder Proteinhydrolysaten und Betain. Da bis auf die kationischen Proteinderivate alle kationischen Polymere bereits zuvor ausführlich beschrieben wurden, sei auf das zuvor Beschriebene verwiesen.

Kationisierte Proteinhydrolysate zählen zu den kationaktiven Substanzen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 250.000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Vorteilhafterweise enthalten die kationisch derivatisierten Proteinhydrolysate eine oder zwei lange C8- bis C22- Alkylketten und entsprechend zwei oder eine kurze C1- bis C4-Alkylketten. Verbindungen, die eine lange Alkylkette enthalten, sind bevorzugt.

Ganz besonders bevorzugt sind die kationischen Proteinhydrolysate und -derivate auf pflanzlicher Basis.

In einer weiteren bevorzugten Ausführungsform kann die Wirkung der erfindungsgemäßen Zusammensetzung durch Emulgatoren (F) gesteigert werden. Emulgatoren bewirken an der Phasengrenzfläche die Ausbildung von wasser-bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994".

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 3 bis 20, gemäß den im Römpp-Lexikon Chemie (Hrg. J. Falbe, M.Regitz), 10. Auflage, Georg

Thieme Verlag Stuttgart, New York, (1997), Seite 1764, aufgeführten Definitionen enthalten. Nichtionogene Emulgatoren mit einem HLB-Wert von 5 - 18 können erfindungsgemäß besonders bevorzugt sein. Ganz besonders bevorzugt können Emulgatoren mit einem HLB - Wert von 10 bis 15 sein.

Weiterhin kann in einer bevorzugten Ausführungsform der Erfindung die Verwendung von UV - Filtern (I) weitere Vorteile bezüglich der Struktur des Haares und damit Vorteile bezüglich der Kämmbarkeit, dem Glanz oder dem Volumen ergeben. Das haar wird mit Hilfe der UV - Filter vor den Einflüssen des UV - Lichtes geschützt. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Bevorzugt sind solche UV-Filter, deren molarer Extinktionskoeffizient am Absorptionsmaximum oberhalb von 15 000, insbesondere oberhalb von 20000, liegt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt. Die Wirkung der erfindungsgemäßen Kombination kann weiterhin durch eine 2-Pyrrolidinon-5-carbonsäure und deren Derivate (J) gesteigert werden. Ein weiterer Gegenstand der Erfindung ist daher die Verwendung des Wirkstoffes in Kombination mit Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Ebenfalls als vorteilhaft hat es erwiesen Vitamine, Provitamine und Vitaminvorstufen sowie deren Derivate (K) den erfindungsgemäßen Zusammensetzungen zuzusetzen. Dabei kann es bevorzugt sein nur solche Vitamine, Provitamine und Vitaminvorstufen und deren Derivate auszuwählen, die nur in Alkohol und/oder Alkohol - Wassergemischen löslich sind.

Dabei sind erfindungsgemäß solche Vitamine, Pro-Vitamine und Vitaminvorstufen bevorzugt, die üblicherweise den Gruppen A, B, C, E, F und H zugeordnet werden.

Bevorzugt enthalten die erfindungsgemäß verwendeten Mittel Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Schließlich läßt sich die Wirkung auch durch den kombinierten Einsatz mit Pflanzenextrakten (L) steigern. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Erfindungsgemäß sind vor allem die Extrakte aus Grünem Tee, Eichenrinde, Brennessel, Hamamelis, Baldrian, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn neben der erfindungsgemäßen Kombination Penetrationshilfsstoffe und/ oder Quellmittel (M) enthalten sind. Diese Hilfsstoffe sorgen für eine bessere Penetration von Wirkstoffen in die keratinische Faser oder helfen die keratinische Faser aufzuquellen. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Der Verbraucher mag bei der Wahrnehmung der Zubereitungen, insbesondere hervorgerufen durch eine ästethische Verpackung, gegebenenfalls in Verbindung mit aromatischen Duftnoten, die erfindungsgemäße Zusammensetzung mit einem Genußmittel in Verbindung bringen. Durch diese Assoziation kann, insbesondere bei Kindern, eine orale Aufnahme bzw. ein Herunterschlucken der Zusammensetzung prinzipiell nicht gänzlich ausgeschlossen werden. In einer bevorzugten Ausführungsform enthalten daher die erfindungsgemäßen Zusammensetzungen einen Bitterstoff, um ein Herunterschlucken bzw. eine akzidentielle Ingestion zu verhindern. Dabei sind erfindungsgemäß Bitterstoffe bevorzugt, die in Wasser bei 20 °C zu mindestens 5 g/l löslich sind.

Hinsichtlich einer unerwünschten Wechselwirkung mit gegebenenfalls in der erfindungsgemäßen Zusammensetzung enthaltenen Duft-Komponenten, insbesondere einer Veränderung der vom Verbraucher wahrgenommenen Duftnote, haben die ionogenen Bitterstoffe sich den nichtionogenen als überlegen erwiesen. Ionogene Bitterstoffe, bevorzugt bestehend aus organischem(n) Kation(en) und organischem(n) Anion(en), sind daher für die erfindungsgemäßen Zubereitungen bevorzugt.

Erfindungsgemäß hervorragend geeignet als Bitterstoffe sind quartäre Ammoniumverbindungen, die sowohl im Kation als auch im Anion eine aromatische Gruppe enthalten. Eine solche Verbindung ist das kommerziell z.B. unter den Warenzeichen Bitrex® und Indige-stin® erhältliche Benzyldiethyl((2,6-Xylylcarbamoyl)methyl)ammoniumbenzoat. Diese Verbindung ist auch unter der Bezeichnung Denatonium Benzoate bekannt.

Der Bitterstoff ist in den erfindungsgemäßen Zusammensetzungen in Mengen von 0,0005 bis 0,1 Gew.-%, bezogen auf den Formkörper, enthalten. Besonders bevorzugt sind Mengen von 0,001 bis 0,05 Gew.-%.

Nicht nur wenn in einer Ausführungsform der Erfindung die Zusammensetzung in Form einer Emulsion vorliegt, können die Emulsion stabilisierende Polymere vorteilhafter Weise verwendet werden. Die Stabilisierung einer Emulsion kann auf verschiedenen Wegen erreicht werden. Beispielsweise können Polymere verwendet werden, welche die Viskosität der Emulsion beeinflussen. Polymere können die Viskosität von wäßrigen und nicht-wäßrigen Phasen in kosmetischen Zubereitungen beeinflussen. In wäßrigen Phasen beruht ihre die Viskosität beeinflussende Funktion auf ihrer Löslichkeit in Wasser oder ihrer hydrophilen Natur. Sie werden sowohl in tensidischen als auch in emulsionsförmigen Systemen angewendet. Wenn die erfindungsgemäße Zusammensetzung in Form einer Emulsion formuliert werden soll, können emulsionsstabilisierende Polymere vorteilhaft als die Viskosität beeinflussende Polymere mitverwendet werden. Hierunter sind Polymere zu verstehen, welche den Aufbau und die Stabilisierung von Emulsionen (O/W und W/O sowie multiple Emulsionen) wesentlich unterstützen. Tenside und Emulgatoren sind selbstverständlich die wesentlichen Bestandteile, jedoch tragen die stabilisierenden Polymere durch eine positive Beeinflussung der kontinuierlichen oder der dispersen Phase zu einer Verringerung der Koaleszenz der emulgierten Tröpfchen bei. Diese positive Beeinflussung kann auf einer elektrischen Abstoßung und einer Erhöhung der Viskosität oder einer Filmbildung auf der Tröpfchenoberfläche beruhen.

Eine weitere Möglichkeit zur Beeinflussung der Viskosität der erfindungsgemäßen Zusammensetzung ist die Verdickung der nicht-wäßrigen Phase, der Lipidphase. Hierzu werden Polymere eingesetzt, welche nicht wasserlöslich aber kompatibel mit Lipiden sind. Sie werden auch zur Gelbildung von kosmetischen Mitteln mit hohen Lipidanteilen verwendet.

Die erfindungsgemäße Kombination umfaßt weiterhin einen Spender, aus welchem das in ihm enthaltene treibgashaltige Haarbehandlungsmittel mit Hilfe des Treibmittels ausgesprüht wird. Erfindungsgemäß umfaßt der Spender ein Ventil, welches eine Ventilöffnung beinhaltet, die als Kegelbohrung ausgestaltet ist und deren Durchmesser 0,25 bis 0,3 mm beträgt. Zusätzlich beinhaltet das Ventil eine Drosselung, welche einen Innendurchmesser von maximal 0,3 mm aufweist, aber besitzt keine Seitenbohrung ("Gasphasenbohrung").

Vorzugsweise beträgt der Durchmesser der Kegelbohrung 0,25 bis 0,3 mm. Vorzugsweise beträgt auch die Tiefe der Kegelbohrung maximal 0,3 mm. Bevorzugte erfindungsgemäße Kombinationen sind weiter dadurch gekennzeichnet, daß die Tiefe der Kegelbohrung 0,1 bis 0,3 mm, vorzugsweise 0,125 bis 0,275 mm, weiter bevorzugt 0,15 bis 0,25 mm und insbesondere 0,2 bis 0,25 mm beträgt.

Die Ausgestaltung des Ventils umfaßt weiterhin eine Drosselung, die den Durchsatz begrenzt. Die Drosselung befindet sich dabei entweder im Stem des Ventiles oder im Sprühkopf. Diese Drosselung weist in bevorzugten Ausführungsformen der vorliegenden Erfindung einen Innendurchmesser von 0,1 bis 0,3 mm, vorzugsweise 0,125 bis 0,275 mm, weiter bevorzugt 0,15 bis 0,25 mm und insbesondere 0,2 bis 0,25 mm auf.

Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten. Auch der Einsatz von Innenbeuteln in Dosen ist problemlos möglich.

Die Dosen sind mit einem geeigneten Sprühkopf ausgestattet. Je nach Sprühkopf sind Ausstoßraten, bezogen auf voll gefüllte Dosen, von 0,1 g/s bis 5,0 g/s möglich. Die Sprührate wird dabei so bestimmt, dass eine mit Treibgas und der entsprechenden Zusammensetzung gefüllte und mit dem betreffenden Ventil verschlossene Aerosoldose bei Raumtemperatur (etwa 23 °C) zunächst gewogen wird. Die Dose wird samt Inhalt 10 mal kräftig von Hand geschüttelt, damit sich der Inhalt gut vermischt. Dann wird für 10 s das Ventil der senkrecht stehenden Dose betätigt. Danach wird wiederum gewogen. Der Vorgang wird 5 mal hintereinander durchgeführt und das statistische Mittel aus den Ergebnissen gebildet. Die Differenz der beiden Wägungen ist die Sprührate pro 10 s. Daraus lässt sich durch einfaches Dividieren die Sprührate je Sekunde bestimmen. Sprühraten von 0,1 bis 0,5 g/s sind dabei bevorzugt. Sprühraten von 0,1 bis 0,4 g/s sind besonders bevorzugt.

Ein weiterer charakteristischer Einfluß für die Effizienz und Formulierbarkeit als Kompaktspray ist das Sprühbild. Das Sprühbild wird durch das Ventil und dessen Beschaffenheit entscheidend beeinflusst. Wenn beispielsweise in einer Haarsprayformulierung der Filmbildner bis auf das fünffache gegenüber einer konventionellen Rezeptur erhöht wird, so sind neben den zu beachtenden und zu vermeidenden erhöhten Viskositäten der Rezeptur auch die bereits diskutierte Sprührate wesentliche für die Formulierung zu beachtende Merkmale. Zusätzlich muß jedoch insbesondere auch das

Sprühbild, das heißt der Öffnungskegel des Ventiles, und die Tröpfchengröße beachtet werden.

Wenn der Öffnungskegel einen zu großen Öffnungswinkel aufweist, dann wird das Produkt bei einem üblichen Abstand der Sprühdose vom Kopf des Anwenders von etwa 10 bis 40 cm auf eine zu kleine Haaroberfläche aufgebracht. Dies führt zu einer Veränderung der Wirksamkeit der Zusammensetzung. Es treten entweder Verklebungen durch zu hohe Produktmengen oder eine zu geringe Festigungswirkung durch eine zu geringe aufgebrachte Produktmenge auf. Im letzteren Falle ist der Öffnungskegel zu groß, so dass eine zu große Haaroberfläche mit der Zusammensetzung behandelt wird. Es hat sich nun gezeigt, dass der Öffnungskegel idealerweise zwischen 25 ° und 65 ° liegen muß. Ein Winkel von 30 ° bis 60 ° ist dabei bevorzugt. Ganz besonders bevorzugt sind Öffnungskegel zwischen 35 ° und 50 °.

Die erfindungsgemäßen Haarbehandlungsmittel zeichnen sich insbesondere dadurch aus, dass die mittlere Tröpfchengröße beim Versprühen vorzugsweise kleiner als 40 μm ist. Vorzugsweise ist die mittlere Teilchengröße kleiner als 38 μm. Überraschenderweise hat sich gezeigt, dass sich die erfindungsgemäßen Haarbehandlungsmittel trotz des hohen Anteils an filmbildendem und/oder festigendem Polymer zuverlässig versprühen lassen, wenn eine entsprechend kleine mittlere Tröpfchengröße vorliegt. Bedingt durch die hohe Wirkstoffkonzentration und die kleine Tröpfchengröße beim Versprühen wird gewährleistet, dass einer Aerosoldose, die das Haarbehandlungsmittel enthält, bei im Vergleich zu üblichen Haarsprays unveränderter Handhabung durch den Verbraucher eine geringere Menge des Mittels entnommen, jedoch der gleiche festigende Effekt erzielt wird.

Die mittlere Tröpfchengröße wird mit einem Laserbeugungsmessgerät vom Typ Masterizer, Series 2600 Droplet and Particle Size Analyzer der Fa. Malvern bestimmt. Hierzu wird die Probe in einem definierten Abstand durch den Lichtstrahl des Lasers gesprüht und anhand der Laserbeugung die Teilchengrößenverteilung bestimmt.

Beispiele:

Es wurde folgende Zusammensetzungen hergestellt (Angaben in Gew.-%):

| | **E** | **V** |
|---|---|---|
| Ultrahold® strong [1] | 9,0 | - |
| Ultrahold® 8 [2] | - | 9,0 |
| 2-Amino-2-methylpropanol | 1,1 | 0,9 |
| Benzophenon-4 | 0,1 | 0,1 |
| Isopropylmyristat | 0,2 | 0,2 |
| PEG/PPG-18/18 DIMETHICONE | 0,03 | 0,03 |
| Ethanol 96%ig | 49,0 | 49,0 |
| Parfüm | 0,05 | 0,05 |
| Dimethylether | 40,0 | 40,0 |
| Wasser | Ad 100 | Ad 100 |

[1] Terpolymer aus N-Butylacrylamid, Ethylacrylat und Acrylsäure (K-Wert gemessen in Anlehnung an DIN EN ISO 1628-1 bei 20°C, 1013,25 mbar in 1 Gew.-%iger Lösung des Polymers in Ethanol: 35 bis 45)
[2] Terpolymer aus N-Butylacrylamid, Ethylacrylat und Acrylsäure (K-Wert gemessen in Anlehnung an DIN EN ISO 1628-1 bei 20°C, 1013,25 mbar in 1 Gew.-%iger Lösung des Polymers in Ethanol: 22 bis 32)

Diese Zusammensetzungen wurden in Druckbehälter verpackt, die ein Ventil mit Kegelbohrung aufwiesen. Der Durchmesser der Kegelbohrung betrug 0,25 mm, die Tiefe der Bohrung betrug 0,3 mm.

Es wurden vier verschiedene Ventiltypen eingesetzt:

- Ventil mit Gasphasenbohrung, ohne Drosselung: MG
- Ventil ohne Gasphasenbohrung, ohne Drosselung: OG
- Ventil mit Gasphasenbohrung und mit Drosselung mit 0,25 mm Innendurchmesser: MGD
- Ventil ohne Gasphasenbohrung und mit Drosselung mit 0,25 mm Innendurchmesser: OGD

Während sich alle Kombinationen der nicht-erfindungsgemäßen Zusammensetzung V mit den vier verschiedenen Ventiltypen problemlos versprühen ließen, bestand bei Kombinationen der erfindungsgemäßen Zusammensetzung E eine Abhängigkeit vom Ventiltyp:

Die nicht erfindungsgemäßen Kombinationen

E + MG (Gasphasenbohrung, keine Drosselung)
E + MGD (Gasphasenbohrung)

zeigten ein unhomogenes Sprühbild. Die Ventile verklebten nach kurzer Zeit, eine verbrauchergerechte Nutzbarkeit bis zur Restentleerung war nicht möglich.

Die nicht erfindungsgemäßen Kombination

E + OG (keine Drosselung)

zeigten ebenfalls ein unhomogenes Sprühbild. Bei gleicher Applikationszeit wurde zuviel Produkt versprüht, so daß die Frisierbarkeit nicht gegeben war und die Haare verklebten, bei verringerter Applikationszeit war eine verbrauchergerechte Verteilung auf dem haar nicht möglich, die Frisierbarkeit war schlecht.

Bei der erfindungsgemäßen Kombination

E + OGD (keine Gasphasenbohrung, Drosselung)

traten diese Probleme nicht auf, das Sprühbild war bis zur Restentleerung der Druckbehälter homogen und fein, die Frisierbarkeit war hervorragend.

## Patentansprüche

1. Kombination aus einem treibmittelhaltigen Haarbehandlungsmittel und einem dafür geeigneten Spender, **dadurch gekennzeichnet, daß**

   A) das treibmittelhaltige Haarbehandlungsmittel bezogen auf sein Gewicht a1

      ) 7 bis 13 Gew.-% eines Terpolymers aus N-Butylacrylamid, Ethylacrylat und Acrylsäure enthält, das einen K-Wert (gemessen in Anlehnung an DIN EN ISO 1628-1 bei 20°C, 1013,25 mbar in 1 Gew.-%iger Lösung des Polymers in Ethanol) von mindestens 35 aufweist,
      a2) mindestens 10 Gew.-% eines oder mehrerer protischer Lösungsmittel enthält,
      a3) 5 bis 60 Gew.-% Treibmittel enthält, und

   B) der Spender ein Ventil umfaßt, welches

      b1) eine Ventilöffnung beinhaltet, die als Kegelbohrung ausgestaltet ist und deren Durchmesser 0,25 bis 0,3 mm beträgt und
      b2) eine Drosselung beinhaltet, welche einen Innendurchmesser von maximal 0,3 mm aufweist,
      b3) keine Seitenbohrung ("Gasphasenbohrung") aufweist.

2. Kombination nach Anspruch 1, **dadurch gekennzeichnet, daß** das treibmittelhaltige Haarbehandlungsmittel bezogen auf sein Gewicht 7,5 bis 12 Gew.-%, besonders bevorzugt 8 bis 11 Gew.-% und insbesondere 8,5 bis 10 Gew.-% mindestens eines Terpolymers aus N-Butylacrylamid, Ethylacrylat und Acrylsäure enthält.

3. Kombination nach einem der Ansprüche 1 oder 2, **dadurch gekennzeichnet, daß** das treibmittelhaltige Haarbehandlungsmittel ein Terpolymer aus N-Butylacrylamid, Ethylacrylat und Acrylsäure enthält, das einen K-Wert (gemessen in Anlehnung an DIN EN ISO 1628-1 bei 20°C, 1013,25 mbar in 1 Gew.-%iger Lösung des Polymers in Ethanol) von 35 bis 60, vorzugsweise von 35 bis 50 und insbesondere von 35 bis 45 aufweist.

4. Kombination nach einem der Ansprüche 1 bis 3, **dadurch gekennzeichnet, daß** das treibmittelhaltige Haarbehandlungsmittel bezogen auf sein Gewicht 10 bis 80 Gew.-%, vorzugsweise 12,5 bis 70 Gew.-%, weiter bevorzugt 15 bis 65 Gew.-%, noch weiter bevorzugt 20 bis 60 Gew.-%, besonders bevorzugt 22,5 bis 55 Gew.-% und insbesondere

25 bis 50 Gew.-% mindestens eines protischen Lösungsmittels, ausgewählt aus Ethanol, Wasser und deren Mischungen, enthält.

5. Kombination nach einem der Ansprüche 1 bis 4, **dadurch gekennzeichnet, daß** das treibmittelhaltige Haarbehandlungsmittel bezogen auf sein Gewicht 7,5 bis 57,5 Gew.-%, vorzugsweise 10 bis 55 Gew.-%, weiter bevorzugt 15 bis 52,5 Gew.-%, noch weiter bevorzugt 17,5 bis 55 Gew.-%, besonders bevorzugt 20 bis 50 Gew.-% und insbesondere 25 bis 45 Gew.-% mindestens eines Treibmittels, ausgewählt aus n-Propan, n-Butan, iso-Butan, n-Pentan, Dimethylether und deren Mischungen, enthält.

6. Kombination nach einem der Ansprüche 1 bis 5, **dadurch gekennzeichnet, daß** die Tiefe der Kegelbohrung 0,1 bis 0,3 mm, vorzugsweise 0,125 bis 0,275 mm, weiter bevorzugt 0,15 bis 0,25 mm und insbesondere 0,2 bis 0,25 mm beträgt.

7. Kombination nach einem der Ansprüche 1 bis 6, **dadurch gekennzeichnet, daß** die Drosselung einen Innendurchmesser von 0,1 bis 0,3 mm, vorzugsweise 0,125 bis 0,275 mm, weiter bevorzugt 0,15 bis 0,25 mm und insbesondere 0,2 bis 0,25 mm aufweist.

## Claims

1. A combination of a propellant-containing hair treatment agent and a dispenser suitable therefor, **characterized in that**

    A) the propellant-containing hair treatment agent contains, based on its weight,

        a1) from 7 to 13 wt.% of a terpolymer of N-butylacrylamide, ethyl acrylate and acrylic acid, which terpolymer has a K value (measured in accordance with DIN EN ISO 1628-1 at 20 °C and at 1013.25 mbar in 1 wt.% solution of the polymer in ethanol) of at least 35,
        a2) at least 10 wt.% of one or more protic solvents,
        a3) from 5 to 60 wt.% of propellant, and

    B) the dispenser comprises a valve which

        b1) includes a valve opening which is designed as a tapered hole and the diameter of which is from 0.25 mm to 0.3 mm and
        b2) includes a restriction that has an inside diameter of at most 0.3 mm,
        b3) has no side hole ("gas phase hole").

2. The combination according to claim 1, **characterized in that** the propellant-containing hair treatment agent contains, based on its weight, from 7.5 to 12 wt.%, particularly preferably from 8 to 11 wt.%, and in particular from 8.5 to 10 wt.%, of at least one terpolymer of N-butylacrylamide, ethyl acrylate and acrylic acid.

3. The combination according to one of claims 1 or 2, **characterized in that** the propellant-containing hair treatment agent contains a terpolymer of N-butylacrylamide, ethyl acrylate and acrylic acid, which terpolymer has a K value (measured in accordance with DIN EN ISO 1628-1 at 20 °C and at 1013.25 mbar in 1 wt.% solution of the polymer in ethanol) of from 35 to 60, preferably from 35 to 50, and in particular from 35 to 45.

4. The combination according to one of claims 1 to 3, **characterized in that** the propellant-containing hair treatment agent contains, based on its weight, from 10 to 80 wt.%, preferably from 12.5 to 70 wt.%, more preferably from 15 to 65 wt.%, even more preferably from 20 to 60 wt.%, particularly preferably from 22.5 to 55 wt.%, and in particular from 25 to 50 wt.%, of at least one protic solvent, selected from ethanol, water and mixtures thereof.

5. The combination according to one of claims 1 to 4, **characterized in that** the propellant-containing hair treatment agent contains, based on its weight, from 7.5 to 57.5 wt.%, preferably from 10 to 55 wt.%, more preferably from 15 to 52.5 wt.%, even more preferably from 17.5 to 55 wt.%, particularly preferably from 20 to 50 wt.%, and in particular from 25 to 45 wt.%, of at least one propellant, selected from n-propane, n-butane, iso-butane, n-pentane, dimethyl ether and mixtures thereof.

6. The combination according to one of claims 1 to 5, **characterized in that** the depth of the tapered hole is from 0.1

to 0.3 mm, preferably from 0.125 to 0.275 mm, more preferably from 0.15 to 0.25 mm, and in particular from 0.2 to 0.25 mm.

7. The combination according to one of claims 1 to 6, **characterized in that** the restriction has an inside diameter of from 0.1 to 0.3 mm, preferably from 0.125 to 0.275 mm, more preferably from 0.15 to 0.25 mm, and in particular from 0.2 to 0.25 mm.

## Revendications

1. Combinaison d'un agent de traitement capillaire contenant un agent propulseur et d'un distributeur approprié à cet effet, **caractérisé en ce que** :

   A) l'agent de traitement capillaire contenant l'agent propulseur contient, rapporté à son poids,

   a1) 7 à 13 % en poids d'un terpolymère issu de l'acrylamide de n-butyle, de l'acrylate d'éthyle et de l'acide acrylique, qui présente une valeur K (mesurée en référence à DIN EN ISO 1628-1 à 20 °C, 1 013,25 mbar dans une solution à 1 % en poids du polymère dans l'éthanol) d'au moins 35,
   a2) au moins 10 % en poids d'un ou de plusieurs agents de solution protique,
   a3) 5 à 60 % en poids d'agent propulseur, et

   B) le distributeur comprend une soupape, laquelle

   b1) contient une ouverture de soupape qui est agencée en tant qu'alésage conique et dont le diamètre est de 0,25 à 0,3 mm et
   b2) contient un étranglement, lequel présente un diamètre interne de 0,3 mm au maximum,
   b3) ne présente aucun alésage latéral (« alésage de phases gazeuses »).

2. Combinaison selon la revendication 1, **caractérisée en ce que** l'agent de traitement capillaire contenant un agent propulseur contient, rapporté à son poids, 7,5 à 12 % en poids, de manière particulièrement préférée 8 à 11 % en poids et en particulier 8,5 à 10 % en poids d'au moins un terpolymère issu de l'acrylamide de n-butyle, de l'acrylate d'éthyle et de l'acide acrylique.

3. Combinaison selon l'une des revendications 1 ou 2, **caractérisée en ce que** l'agent de traitement capillaire contenant un agent propulseur contient un terpolymère issu de l'acrylamide de n-butyle, de l'acrylate d'éthyle et de l'acide acrylique, qui présente une valeur K (mesurée en référence à DIN EN ISO 1628-1 à 20 °C, 1 013,25 mbar dans une solution à 1 % en poids du polymère dans l'éthanol) de 35 à 60, de préférence de 35 à 50 et en particulier de 35 à 45.

4. Combinaison selon l'une des revendications 1 à 3, **caractérisée en ce que** l'agent de traitement capillaire contenant un agent propulseur contient, rapporté à son poids, 10 à 80 % en poids, de préférence 12,5 à 70 % en poids, de manière davantage préférée 15 à 65 % en poids, de manière encore davantage préférée 20 à 60 % en poids, de manière particulièrement préférée de 22,5 à 55 % en poids et en particulier de 25 à 50 % en poids d'au moins un agent de solution protique, sélectionné parmi l'éthanol, l'eau, ainsi que leurs mélanges.

5. Combinaison selon l'une des revendications 1 à 4, **caractérisée en ce que** l'agent de traitement capillaire contenant un agent propulseur contient, rapporté à son poids, 7,5 à 57,5 % en poids, de préférence 10 à 55 % en poids, de manière davantage préférée 15 à 52,5 % en poids, de manière encore davantage préférée 17,5 à 55 % en poids, de manière particulièrement préférée 20 à 50 % en poids et en particulier 25 à 45 % en poids d'au moins un agent propulseur, sélectionné parmi le n-propane, le n-butane, l'isobutane, le n-pentane, l'éther diméthylique, ainsi que leurs mélanges.

6. Combinaison selon l'une des revendications 1 à 5, **caractérisé en ce que** la profondeur de l'alésage conique est de 0,1 à 0,3 mm, de préférence de 0,125 à 0,275 mm, de manière davantage préférée de 0,15 à 0,25 mm et en particulier de 0,2 à 0,25 mm.

7. Combinaison selon l'une des revendications 1 à 6, **caractérisée en ce que** l'étranglement présente un diamètre interne allant de 0,1 à 0,3 mm, de préférence de 0,125 à 0,275 mm, de manière davantage préférée de 0,15 à 0,25

mm et en particulier de 0,2 à 0,25 mm.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005018588 A2
- DE PS4413686 C
- GB 2104091 A
- EP 47714 A
- EP 217274 A
- EP 283817 A
- DE 2817369
- DE 3929973
- EP 0612759 B1
- DE OS19756454 A


### In der Beschreibung aufgeführte Nicht-Patentliteratur


- International Cosmetic Ingredient Dictionary and Handbook. The Cosmetic, Toiletry, and Fragrance Association, 1997
- **H.-D.DÖRFLER.** Grenzflächen- und Kolloidchemie. VCH Verlagsgesellschaft mbH, 1994
- Römpp-Lexikon Chemie. Georg Thieme Verlag, 1997, 1764